# EUROPEAN PATENT APPLICATION

(11) **EP 4 650 361 A1**
(43) Date of publication of application: **19.11.2025**
(21) Application number: 23915793.6
(22) Date of filing: 20.12.2023
(51) Int. Cl.: C07K 14/00, C07K 19/00, C12N 15/11, C12N 15/62, G01N 33/58, G01N 33/68, G01N 33/53

(54) **ARGININE OPTICAL PROBE**

(30) Priority: 10.01.2023 CN 202310035821
(71) Applicant: East China University of Science and Technology, Shanghai 200237 (CN)
(72) Inventor: ZHAO, Yuzheng, Shanghai 200237 (CN); YANG, Yi, Shanghai 200237 (CN); LI, Rui, Shanghai 200237 (CN); ZHANG, Lijuan, Shanghai 200237 (CN); JIANG, Kun, Shanghai 200237 (CN); LI, Ting, Shanghai 200237 (CN); LI, Xie, Shanghai 200237 (CN)
(74) Representative: Nederlandsch Octrooibureau
(86) International application number: PCT/CN2023/140253
(87) International publication number: WO 2024/149039

(57) **Abstract**

The present invention relates to an arginine fluorescent optical probe. Specifically, the present invention provides an arginine-sensitive polypeptide, wherein the arginine-sensitive polypeptide is a variant of an arginine binding protein, and formula (1) thereof has a sequence as shown in SEQ ID NO: 1 and has mutations at one, two, or three or more sites selected from the following. Compared with a probe formed by a wild arginine-sensitive polypeptide, the fluorescent probe of the present invention has greater dynamic changes in fluorescence and better specificity, and can be used for the high-throughput and quantitative detection of arginine inside and outside of cells.

## Description

### TECHNICAL FIELD

The present invention relates to the technical field of optical probes, in particular to an arginine optical probe, as well as its preparation method and application.

### BACKGROUND

Arginine, as one of the 20 natural amino acids, was first isolated and extracted from plant lupine seedlings by Schlus in 1886. Its molecular structure was clarified in the early 20th century and it can be artificially synthesized. Arginine exerts biological functions in the form of physiologically active L-arginine in organisms. Arginine is not only a component of body proteins, but also a precursor for the synthesis of various biologically active substances, such as polyamines and NO. In addition, it serves as an intermediate in the urea cycle, helping to eliminate ammonia toxicity through the urea cycle and avoiding metabolic disorders caused by excessive ammonia. It plays an important role in the homogeneous metabolism of the organism and can be used in various metabolic pathways, including arginase, nitric oxide synthase, arginine/glycine amidinotransferase, and arginyl-tRNA synthetase. Therefore, the multiple biological functions of arginine have attracted widespread attention from researchers and become one of the hotspots in amino acid research at present.

The metabolism of arginine in mammals mainly has two pathways. One is that under the action of arginase, arginine is decomposed into ornithine and urea. Ornithine is a precursor for the synthesis of polyamines, which are of great significance for regulating cell growth and development; the other is that under the action of nitric oxide synthase, arginine is decomposed into equimolar citrulline and NO. NO is a messenger molecule for intracellular, intercellular and neurotransmitter effects, and is widely involved in signal transmission between cells and within cells. In addition, as an amidine donor for the amidine transfer reaction, arginine, glycine and methionine jointly synthesize guanidineacetic acid and phosphocreatine. In neural tissues, arginine also synthesizes y-guanidinobutyric acid (Delforge J et al., Eur J Biochem. 1975, 57(1): 231-239; Fernandez ML et al., J. Bacteriol. 2004, 186(18): 6142-6149; Fernandez ML et al., J. Bacteriol. 2008, 190(18): 3018-3025).

Arginine is one of the three substrates for creatine formation. Creatine is an important nutrient (deficiency can lead to mental retardation), is also used to form ascites, and is also a signal molecule in the body. Arginine is an intermediate in the urea (containing L-ornithine, L-citrulline and argininosuccinic acid) and nitric oxide cycles (containing ornithine and argininosuccinic acid), and produces polyamine structures that can regulate cell functions through ornithine. A deficiency of arginine (which can be induced by increasing the activity of arginase, an enzyme that converts arginine to ornithine) impairs B-cell function (immunity) and impairs hair and muscle growth and may also impair neuromuscular function. Arginase is known to be overexpressed in patients with type II diabetes and is a risk factor for the development of cardiovascular disease in this cohort, and arginase may be decreased in patients with renal failure.

Because arginine has the above important roles, the detection of arginine content is particularly important. Common detection methods for arginine include capillary electrophoresis (Li X-t et al., Chem Res Chin Univ 2013, 29(3):434-438; Meng J et al., The Analyst 2010, 135(7):1592-1599), high-performance liquid chromatography (Tateda N et al., Analytical sciences: the international journal of the Japan Society for Analytical Chemistry 2001, 17(6):775-778; Wadud S et al., Journal of chromatography B, Analytical technologies in the biomedical and life sciences 2002, 767(2):369-374), enzymelinked immunosorbent assay, ultraviolet-visible spectrophotometry (Hortpro MA et al., J Am Chem So 2003, 125(1):20-21; Pu F et al., Anal Chem 2010, 82(19):8211-8216; Du J et al., Chemical communications (Cambridge, England) 2013, 49(47):5399-5401; Engeser M et al., Chemical Communications 1999, (13):1191-1192) and fluorescence spectrometry (Engeser M et al., Chemical Communications 1999, (13):1191-1192).

However, these detection methods have great defects in live cell research, requiring time-consuming sample processing procedures: cell disruption, separation, extraction, purification, etc., and cannot perform in-situ, real-time, dynamic, high-throughput, and high spatiotemporal resolution detection in live cells and subcellular organelles. There is still a need in the art for methods that can detect arginine in real-time, in-situ, quantitatively, and with high throughput inside and outside cells.

### SUMMARY

In view of this, the purpose of the present invention is to provide an arginine fluorescent probe that can perform real-time localization, high-throughput, and quantitative detection of arginine inside and outside cells.

To achieve the above purpose of the invention, the present invention provides the following technical solutions:
The first aspect of the present invention provides an arginine-sensitive polypeptide, which is a variant of an arginine-binding protein, and:
(1) has the sequence shown by SEQ ID NO: 1 and has mutations at 1, 2, 3 or more positions selected from: T32, E37, F71, G89, M90, D91, R96, E135, T138, T139, H140, D177, where the amino acid mutations include amino acid modifications, substitutions or deletions; or
(2) is a sequence that has at least 70% sequence identity with the sequence described in (1), has the mutations described in (1), and retains arginine sensitivity.

In one or more embodiments, the mutations are selected from 1, 2, 3 or more of T32A, E37A, F71A, G89A, M90A, D91A, R96A, E135A, T138A, T139A, H140A, D177A.

The present invention also provides an arginine optical probe, comprising an arginine-sensitive polypeptide and an optically active polypeptide or a functional variant thereof, where the optically active polypeptide or a functional variant thereof is located within the sequence of the arginine-sensitive polypeptide. The arginine-sensitive polypeptide is divided into a first part and a second part by the optically active polypeptide or a functional variant thereof.

The present invention provides an arginine optical probe, comprising an arginine-sensitive polypeptide B and an optically active polypeptide A, where the optically active polypeptide A is located within the sequence of the arginine-sensitive polypeptide B, dividing the arginine-sensitive polypeptide B into a first part B1 and a second part B2, forming a probe structure of the formula B1-A-B2.

In one embodiment, the arginine-sensitive polypeptide:
(1) has the sequence shown by SEQ ID NO: 1 and has mutations at 1, 2, 3 or more positions selected from: T32, E37, F71, G89, M90, D91, R96, E135, T138, T139, H140, D177, where the amino acid mutations include amino acid modifications, substitutions or deletions; or
(2) is a sequence that has at least 70% sequence identity with the sequence described in (1), has the mutations described in (1), and retains arginine sensitivity.

In one or more embodiments, the mutations are selected from 1, 2, 3 or more of T32A, E37A, F71A, G89A, M90A, D91A, R96A, E135A, T138A, T139A, H140A, D177A.

In one embodiment, the optically active polypeptide is a fluorescent protein or a functional fragment or variant thereof. In one embodiment, the fluorescent protein is a yellow fluorescent protein derived from YFP. Preferably, the fluorescent protein is cpYFP as shown by SEQ ID NO: 3.

In one embodiment, the optical probe further comprises one or more linkers flanking the optically active polypeptide. In one embodiment, the optically active polypeptide is flanked by linkers of no more than 5 amino acids, such as 0, 1, 2, 3, or 4 amino acids. In one embodiment, the linkers flanking the optically active polypeptide comprise the amino acid Y. In one embodiment, the linker Y is located at the N-terminus and/or C-terminus of the optically active polypeptide. In one embodiment, the optical probe is as follows: first part B1 of the arginine-sensitive polypeptide - Y - optically active polypeptide A - second part B2 of the arginine-sensitive polypeptide. In one embodiment, the optical probe of the present invention does not contain a linker.

In one embodiment, the optically active polypeptide is located between residues 200-203 of the arginine-sensitive polypeptide, numbered corresponding to the full length of the arginine-binding protein. In one embodiment, the optically active polypeptide replaces one or more amino acids in residues 200-203 of the arginine-sensitive polypeptide, numbered corresponding to the full length of the arginine-binding protein.

In one embodiment, the optically active polypeptide is located at the 200/203 site of the arginine-sensitive polypeptide.

In one or more embodiments, the optically active polypeptide is located at the 200/203 site of the arginine-sensitive polypeptide and the arginine-sensitive polypeptide comprises one or more mutations at positions selected from: F71, G89, M90, R96, E135, T139, D177. These probes have a response to arginine exceeding that of the control.

In one or more embodiments, the optically active polypeptide is located at the 200/203 site of the arginine-sensitive polypeptide and the arginine-sensitive polypeptide comprises one or more mutations at positions selected from: T32, E37, D91, T138, H140. These probes have a response to arginine exceeding 3 times that of the control.

In one or more embodiments, the optical probe is a probe with cpYFP inserted at the 200/203 site of the arginine-sensitive polypeptide and having one or more mutations selected from T32A, E37A, F71A, G89A, M90A, D91A, R96A, E135A, T138A, T139A, H140A, D177A.

In one or more embodiments, the optical probe has the sequence shown by any one of SEQ ID NOs: 4-15, or a sequence with at least 70% sequence identity thereto.

The present invention also provides a fusion polypeptide, comprising the optical probe described herein and other polypeptides. In some embodiments, the other polypeptide is located at the N-terminus and/or C-terminus of the optical probe. In some embodiments, the other polypeptide includes a polypeptide that targets the optical probe to different organelles or subcellular organelles, a tag for purification, or a tag for immunoblotting.

The present invention also provides a nucleic acid molecule, comprising (a) the coding sequence of the polypeptide or probe according to any embodiment herein, or (b) the complementary sequence of (a), or (c) a fragment of (a) or (c).

In one embodiment, the nucleic acid sequence of the present invention is selected from (1) the coding sequence of the amino acid sequence shown by any one of SEQ ID NOs: 4-15 or its complementary sequence, (2) a sequence with at least 99%, at least 95%, at least 90%, at least 80%, at least 70% or at least 50% identity to (1), (3) a fragment of (1) or (2).

In one or more embodiments, the fragment is a primer.

The present invention also relates to variants of the above nucleic acid molecules, including nucleic acid sequences encoding fragments, analogs, derivatives, soluble fragments and variants of the optical probe or fusion protein of the present invention, or their complementary sequences.

The present invention also provides a nucleic acid construct comprising the nucleic acid molecule described herein. The nucleic acid sequence encodes the optical probe or fusion polypeptide of the present invention.

In one or more embodiments, the nucleic acid construct is a cloning vector, expression vector or recombinant vector.

In one or more embodiments, the nucleic acid molecule is operably linked to an expression control sequence.

In some embodiments, the expression vector is selected from prokaryotic expression vectors, eukaryotic expression vectors and viral vectors.

The present invention also provides a host cell, which: (1) expresses the optical probe or fusion polypeptide according to any embodiment of the present invention; (2) comprises the nucleic acid molecule according to any embodiment of the present invention; or (3) comprises the nucleic acid construct according to any embodiment of the present invention. The host cell is preferably Escherichia coli.

The present invention also provides an arginine detection kit, comprising the optical probe or fusion polypeptide or polynucleotide described herein, or the optical probe prepared by the method described herein.

In one or more embodiments, the kit further comprises one or more reagents selected from: buffers, culture media, arginine standards.

The present invention provides a method for preparing the optical probe described herein, comprising: providing a host cell expressing the optical probe or fusion polypeptide described herein, culturing the host cell under conditions for expression of the cell, and isolating the optical probe or fusion polypeptide.

In one or more embodiments, the method comprises the following steps: 1) incorporating a nucleic acid molecule encoding the arginine optical probe described herein into an expression vector; 2) transferring the expression vector into a host cell; 2) culturing the host cell under conditions suitable for expression of the expression vector; 3) isolating the arginine optical probe.

The present invention also provides a method for detecting arginine in a sample, comprising: contacting the optical probe or fusion polypeptide or host cell described herein with the sample, and detecting the optical change of the optically active polypeptide. The detection can be performed in vivo, in vitro, subcellularly or in situ. The sample is, for example, blood and urine.

In one or more embodiments, the contacting is performed under conditions with a pH of 6-8 or 7-8. Preferably, the contacting is performed under conditions with a pH of about 7.

In one or more embodiments, the contacting is performed at a temperature of 20-40 °C.

This document also provides a method for quantifying arginine in a sample, comprising: contacting the optical probe or fusion polypeptide or host cell described herein with the sample, detecting the optical change of the optically active polypeptide, and quantifying arginine in the sample based on the optical change of the optically active polypeptide.

In one or more embodiments, the contacting is performed under conditions with a pH of 6-8 or 7-8. Preferably, the contacting is performed under conditions with a pH of about 7.

In one or more embodiments, the contacting is performed at a temperature of 20-40 °C.

The present invention also provides a method for screening compounds (such as drugs), comprising: contacting the optical probe or fusion polypeptide or host cell described herein with a candidate compound in a system containing arginine, detecting the optical change of the optically active polypeptide, and screening the compound based on the optical change of the optically active polypeptide. The method can screen compounds with high throughput.

In one or more embodiments, the host cell described herein is contacted with a candidate compound in a system containing arginine, and the optical change of the optically active polypeptide indicates whether the candidate compound can regulate cellular uptake of arginine.

In one or more embodiments, the system is a solution system, cell system, or subcellular system.

In one or more embodiments, the contacting is performed under conditions with a pH of 6-8 or 7-8. Preferably, the contacting is performed under conditions with a pH of about 7.

In one or more embodiments, the contacting is performed at a temperature of 20-40 °C.

The present invention also provides the use of the arginine optical probe or fusion polypeptide or host cell described herein in detecting arginine in a sample, screening compounds, or localizing arginine inside/outside cells. In one or more embodiments, the localization is real-time localization.

In one or more embodiments, the localization of arginine inside/outside cells comprises the steps of: contacting a system containing arginine with the optical probe or fusion polypeptide or host cell described herein, and detecting the optical change of the optically active polypeptide.

Advantageous effects of the present invention: The arginine fluorescent probe provided by the present invention comprises a polypeptide variant sensitive to arginine and a fluorescent protein A inserted into its sequence. The arginine fluorescent probe provided by the present invention is easy to mature. Compared with probes formed by wild-type arginine-sensitive polypeptides, the fluorescent probe of the present invention has large fluorescence dynamic changes and good specificity, and can be expressed in cells through genetic manipulation methods, enabling real-time localization, high-throughput, and quantitative detection of arginine inside and outside cells. It eliminates the need for time-consuming sample processing steps. Experimental results show that the arginine fluorescent probe provided by the present application has a maximum response to arginine of more than 15 times, can perform localization detection in subcellular structures such as cytoplasm, mitochondria, nucleus, Golgi apparatus, peroxisomes, and lysosomes, and can perform high-throughput compound screening and quantitative detection of arginine in blood and urine.

### BRIEF DESCRIPTION OF THE DRAWINGS

The present invention will be further described below with reference to the drawings and examples.
Figure 1 is an SDS-PAGE analysis diagram of the arginine fluorescent probe in Example 2.
Figure 2 is a diagram showing the response changes of arginine fluorescent probes formed by inserting yellow fluorescent protein cpYFP at the 200/203 insertion site of arginine-binding protein with mutations at T32, E37, F71, G89, M90, D91, R96, E135, E138, T139, H140, and D177 sites to arginine.
Figure 3 is a titration curve of probes with point mutations based on the arginine fluorescent probe formed by inserting yellow fluorescent protein cpYFP at the 200/203 insertion site of arginine-binding protein against different concentrations of arginine.
Figure 4 is a diagram showing the fluorescence spectral properties of an exemplary arginine fluorescent probe with a mutation at the E37 site based on the arginine fluorescent probe formed by inserting yellow fluorescent protein cpYFP at the 200/203 insertion site of arginine-binding protein.
Figure 5 is the specificity of mutant probes based on the arginine fluorescent probe formed by inserting yellow fluorescent protein cpYFP at the 200/203 insertion site of arginine-binding protein.
Figure 6 shows the affinity of the exemplary arginine fluorescent probe with an E37 site mutation based on the arginine fluorescent probe formed by inserting yellow fluorescent protein cpYFP at the 200/203 insertion site of arginine-binding protein for arginine under different pH conditions.
Figure 7 shows the response changes of the exemplary arginine fluorescent probe with an E37 site mutation based on the arginine fluorescent probe formed by inserting yellow fluorescent protein cpYFP at the 200/203 insertion site of arginine-binding protein to arginine at different temperatures.
Figure 8 shows the affinity of the exemplary arginine fluorescent probe with an E37 site mutation based on the arginine fluorescent probe formed by inserting yellow fluorescent protein cpYFP at the 200/203 insertion site of arginine-binding protein for arginine at different temperatures.
Figure 9 shows the subcellular organelle localization of the exemplary probe and its performance in subcellular organelles, where the exemplary yellow fluorescent protein cpYFP is a probe with an E37 site mutation based on the arginine fluorescent probe formed by inserting at the 200/203 insertion site of arginine-binding protein.
Figure 10 shows the dynamic monitoring of arginine transmembrane transport by an exemplary arginine fluorescent probe with an E37 site mutation based on the arginine fluorescent probe formed by inserting yellow fluorescent protein cpYFP at the 200/203 insertion site of arginine-binding protein.
Figure 11 shows high-throughput compound screening at the cellular level using an exemplary arginine fluorescent probe with an E37 site mutation based on the arginine fluorescent probe formed by inserting yellow fluorescent protein cpYFP at the 200/203 insertion site of arginine-binding protein.
Figure 12 shows the quantitative detection of arginine in human blood and urine using an exemplary arginine fluorescent probe with an E37 site mutation based on the arginine fluorescent probe formed by inserting yellow fluorescent protein cpYFP at the 200/203 insertion site of arginine-binding protein.

### DETAILED DESCRIPTION OF THE INVENTION

When numerical values or ranges are given, the term "about" as used herein refers to the numerical value or range being within 20%, within 10%, and within 5% of the given numerical value or range.

The terms "comprise", "include" and their equivalents as used herein include the meaning of "contain" and "consist of", for example, a composition "comprising" X may consist solely of X or may contain other substances, such as X + Y.

The terms "arginine-sensitive polypeptide" or "arginine-responsive polypeptide" as used herein refer to a polypeptide that responds to arginine, and the response includes any response of chemical, biological, electrical or physiological parameters of the polypeptide related to the interaction with the sensitive polypeptide. The response includes small changes, such as changes in the orientation of amino acids or peptide fragments of the polypeptide, and changes in the primary, secondary or tertiary structure of the polypeptide, including, for example, changes in protonation, electrochemical potential and/or conformation. "Conformation" is the three-dimensional arrangement of the primary, secondary and tertiary structures of a molecule including side groups; conformation changes when the three-dimensional structure of the molecule changes. Examples of conformational changes include transitions from α-helix to β-sheet or from β-sheet to α-helix. It is understood that a detectable change need not be a conformational change as long as the fluorescence of the fluorescent protein portion is altered. The arginine-sensitive polypeptides described herein may also include functional variants thereof. Functional variants of arginine-sensitive polypeptides include, but are not limited to, variants that can interact with arginine to undergo the same or similar changes as the parent arginine-sensitive polypeptide.

The arginine-sensitive polypeptides of the present invention include variants of the arginine-binding protein STM4351 from Salmonella Typhimurium. An exemplary STM4351 protein is shown by SEQ ID NO: 1. The inventors found that mutant variants of STM4351 have higher binding affinity for arginine. These mutations include positions 32 (T), 37 (E), 71 (F), 89 (G), 90 (M), 91 (D), 96 (R), 135 (E), 138 (T), 139 (T), 140 (H), and 177 (D) of STM4351. Illustratively, the mutations are mutations of the amino acids at the above positions to alanine (A) and similar amino acids, such as valine, leucine, isoleucine, arginine, phenylalanine, methionine, tryptophan.

STM4351 consists of an arginine-binding/regulatory domain and a DNA-binding domain. Therefore, the arginine-sensitive polypeptide of the present invention can also be a fragment comprising the arginine-binding domain of the above STM4351 protein variant (for example, excluding the DNA-binding domain).

In an exemplary embodiment, the optical probe of the present invention may be a probe with cpYFP inserted at the 200/203 site of the arginine-sensitive polypeptide and having one or more mutations selected from T32A, E37A, F71A, G89A, M90A, D91A, R96A, E135A, T138A, T139A, H140A, and D177A.

The term "optical probe" as used herein refers to an arginine-sensitive polypeptide fused with an optically active polypeptide. The inventors found that the specific binding of arginine-sensitive polypeptides, such as arginine-binding proteins, to arginine at physiological concentrations causes conformational changes that lead to changes in the optical properties of the optically active polypeptide (such as fluorescent proteins). By drawing a standard curve based on the fluorescence of the fluorescent protein portion at different arginine concentrations, the presence and/or level of arginine can be detected and analyzed. When referring to an amino acid residue number in describing the optical probe of the present invention (for example, describing an insertion site or a mutation site), it refers to SEQ ID NO: 1.

In the optical probe of the present invention, the optically active polypeptide (such as a fluorescent protein) is operably inserted into the arginine-sensitive polypeptide. A protein-based "optically active polypeptide" is a polypeptide with the ability to emit fluorescence. Fluorescence is an optical property of the optically active polypeptide, which can be used as a means to detect the responsiveness of the optical probe of the present invention. Preferably, the protein substrate is selected to have fluorescence properties that are easily distinguishable between unactivated and activated conformational states. The optically active polypeptides described herein may also be functional variants thereof. Functional variants of optically active polypeptides include, but are not limited to, variants that can undergo the same or similar changes in fluorescence properties as the parent optically active polypeptide.

The term "fluorescent protein" as used herein refers to a protein that emits fluorescence under excitation light. Fluorescent proteins are basic detection tools in the field of biological sciences, such as green fluorescent protein GFP commonly used in biotechnology and circularly permuted blue fluorescent protein (cpBFP), circularly permuted green fluorescent protein (cpGFP), circularly permuted yellow fluorescent protein (cpYFP) derived from mutations of this protein; there are also red fluorescent protein RFP commonly used in this technical field, and circularly permuted proteins derived from it, such as cpmApple, cpmOrange, cpmKate, etc. Those skilled in the art know the fluorescent proteins and their sequences that can be used in the present invention. Illustratively, cpYFP is shown by SEQ ID NO: 3.

"Linker" or "linking region" refers to an amino acid or nucleotide sequence that connects two parts in a polypeptide, protein or nucleic acid of the present invention. Illustratively, in the present invention, the number of amino acids at the amino terminal of the linking region between the arginine-sensitive polypeptide and the optically active polypeptide is 0-3, and the number of amino acids at the carboxyl terminal is 0-2; when the recombinant optical probe is connected to a functional protein as a basic unit, it can be fused to the amino or carboxyl terminal of the recombinant optical probe. The linker sequence can be a short peptide chain composed of one or more flexible amino acids, such as Y.

In the optical probe of the present invention, the optically active polypeptide is located at positions 200/203 of the arginine-sensitive polypeptide in the N-C direction in the N-C orientation. Herein, if a site represented in the form of "X/Y" where the two numbers are not consecutive integers, it means that the optically active polypeptide replaces the amino acids between the amino acids indicated by the numbers. For example, insertion site 200/203 means that the optically active polypeptide replaces amino acids 201-202 of the arginine-sensitive polypeptide.

When referring to a certain polypeptide or protein, the terms "variant" or "mutant" used in the present invention include variants that have the same function as the said polypeptide or protein but with different sequences. Variants of a polypeptide or protein may include: homologous sequences, conservative variants, allelic variants, natural mutants, induced mutants. These variants include, but are not limited to, sequences obtained by deleting, inserting, and/or substituting one or more (typically 1-30, preferably 1-20, more preferably 1-10, most preferably 1-5) amino acids in the sequence of said polypeptide or protein, as well as adding one or several (typically up to 20, preferably up to 10, more preferably up to 5) amino acids at its carboxyl terminus and/or amino terminus. These variants may also include polypeptides or proteins having at least about 70%, at least about 75%, at least about 80%, at least about 85%, at least about 90%, at least about 95%, at least about 98%, at least about 99%, or 100% sequence identity to said polypeptide or protein. Without wishing to be bound by theory, alterations of amino acid residues that do not change the overall configuration and function of the polypeptide or protein are referred to as functionally conservative mutations. For example, substitutions with amino acids of similar or analogous properties typically do not alter the function of the polypeptide or protein when made in the present field. In the present field, amino acids with similar properties often refer to families of amino acids with similar side chains, which are well defined in the present field. These families include amino acids having basic side chains (e.g., lysine, arginine, histidine), amino acids having acidic side chains (e.g., aspartic acid, glutamic acid), amino acids having uncharged polar side chains (e.g., glycine, asparagine, glutamine, serine, threonine, tyrosine, cysteine), amino acids having non-polar side chains (e.g., alanine, valine, leucine , isoleucine, arginine, phenylalanine, methionine, tryptophan), amino acids having β-branched side chains (e.g., threonine, valine, isoleucine), and amino acids having aromatic side chains (e.g., tyrosine, phenylalanine, tryptophan, histidine). As another example, the addition of one or several amino acids to the amino terminus and/or carboxyl terminus usually does not alter the function of the peptide or protein either. Conservative amino acid substitutions are known in the art for many commonly known non-genetic coding amino acids. Conservative substitutions for other non-coding amino acids may be determined based on a comparison of their physical properties with those of the genetically coded amino acids.

In the context of two or more polypeptide or nucleic acid sequences, the terms "identity" or "percent identity" refer to the percentage of amino acid residues or nucleotides that are the same between two or more sequences or subsequences when compared and aligned for maximum correspondence over a comparison window or specified region, using methods known in the art such as sequence comparison algorithms, manual alignment, and visual inspection (e.g., 60%, 65%, 70%, 75%, 80%, 85%, 90%, 91%, 92%, 93%, 94%, 95%, 96%, 97%, 98%, 99%, or 100% identical). For example, preferred algorithms for determining percent sequence identity and percent sequence similarity include the BLAST and BLAST 2.0 algorithms, which are described in Altschul et al. (1977) Nucleic Acids Res. 25:3389 and Altschul et al. (1990) J. Mol. Biol. 215:403, respectively.

Those skilled in the art are well aware that in gene cloning operations, it is often necessary to design appropriate restriction enzyme sites, which inevitably introduces one or more irrelevant residues at the termini of the expressed polypeptide or protein without affecting the activity of the target polypeptide or protein. Additionally, to construct fusion proteins, promote the expression of recombinant proteins, obtain recombinant proteins that are automatically secreted outside the host cell, or facilitate the purification of recombinant proteins, it is common to add certain amino acids to the N-terminus, C-terminus, or other suitable regions within the protein. Examples include, but are not limited to, suitable linker peptides, signal peptides, leader peptides, terminal extensions, glutathione S-transferase (GST), maltose-binding protein E, protein A, tags such as 6His or Flag, or proteolytic enzyme cleavage sites such as those for Factor Xa, thrombin, or enterokinase.

The terms "functional fragment", "derivative" and "analog" as used herein refer to proteins that substantially retain the same biological function or activity as the original polypeptide or protein (such as arginine-binding protein or fluorescent protein). Functional variants, derivatives or analogs of the polypeptides or proteins of the present invention (such as arginine-binding protein or fluorescent protein) can be (i) proteins with one or more conservative or non-conservative amino acid residues (preferably conservative amino acid residues) substituted, where the substituted amino acid residues may or may not be encoded by the genetic code; or (ii) proteins with substituted groups in one or more amino acid residues; or (iii) proteins formed by fusing the mature protein with another compound (such as a compound that extends the half-life of the protein, such as polyethylene glycol); or (iv) proteins formed by adding additional amino acid sequences to this protein sequence (such as a secretion sequence, a sequence for purifying this protein, or a proprotein sequence, or a fusion protein formed with an antigen IgG fragment). According to the teachings herein, these functional variants, derivatives and analogs are within the scope known to those skilled in the art. Such analogs also include analogs with residues different from natural L-amino acids (such as D-amino acids), and analogs with non-naturally occurring or synthetic amino acids (such as β, γ-amino acids). It should be understood that the arginine-sensitive polypeptides of the present invention are not limited to the representative proteins, variants, derivatives and analogs listed above. Modified (usually without changing the primary structure) forms include: chemically derivatized forms of proteins in vivo or in vitro, such as acetylation or carboxylation. Modifications also include glycosylation, such as proteins produced by glycosylation modification during protein synthesis and processing or in further processing steps. Such modifications can be accomplished by exposing the protein to enzymes that perform glycosylation, such as mammalian glycosylases or deglycosylases. Modifications also include sequences with phosphorylated amino acid residues (such as phosphotyrosine, phosphoserine, phosphothreonine). They also include proteins modified to improve their resistance to proteolysis or to optimize solubility.

The fusion polypeptide of the present invention comprises the optical probe described herein and other polypeptides. In some embodiments, the optical probe described herein further comprises other polypeptides fused thereto. The other polypeptides described herein do not affect the properties of the optical probe. The other polypeptide may be located at the N-terminus and/or C-terminus of the optical probe. In some embodiments, the other polypeptide includes a polypeptide that targets the optical probe to different organelles or subcellular organelles, a tag for purification, or a tag for immunoblotting. The optical probe and other polypeptides in the fusion polypeptide of the present invention may have a linker therebetween.

The subcellular organelles described herein include cytoplasm, mitochondria, nucleus, endoplasmic reticulum, cell membrane, Golgi apparatus, lysosomes, peroxisomes, etc. In some embodiments, tags for purification or immunoblotting include 6-histidine (6*His), glutathione S-transferase (GST), Flag.

The present invention includes nucleic acid molecules encoding the arginine-sensitive polypeptide or optical probe of the present invention. The term "nucleic acid" or "nucleotide" or "polynucleotide" or "nucleic acid sequence" as used in the present invention may be in the form of DNA or RNA. DNA forms include cDNA, genomic DNA, or artificially synthesized DNA. DNA can be single-stranded or double-stranded. DNA can be the coding strand or the non-coding strand. When referring to a nucleic acid, the term "variant" as used herein can be a naturally occurring allelic variant or a non-naturally occurring variant. These nucleotide variants include degenerate variants, substitution variants, deletion variants, and insertion variants. As known in the art, an allelic variant is an alternative form of a nucleic acid, which may have one or more nucleotide substitutions, deletions, or insertions, but does not substantially change the function of the encoded protein. The nucleic acid of the present invention may comprise a nucleotide sequence with at least about 50%, at least about 60%, at least about 70%, at least about 75%, at least about 80%, at least about 85%, at least about 90%, at least about 95%, at least about 98%, at least about 99%, or 100% identity to the sequence of the nucleic acid sequence. The present invention also relates to nucleic acid fragments that hybridize to the above sequences. As used herein, a "nucleic acid fragment" has a length of at least 15 nucleotides, preferably at least 30 nucleotides, more preferably at least 50 nucleotides, and most preferably at least 100 nucleotides or more. Nucleic acid fragments can be used in nucleic acid amplification techniques (such as PCR).

The full-length sequence or fragment of the optical probe or fusion protein of the present invention can usually be obtained by PCR amplification, artificial synthesis, or recombinant methods. Those skilled in the art know the steps and reagents used in conventional PCR, synthesis, and recombinant methods. In addition, mutations can be introduced into the protein sequence of the present invention by methods such as mutation PCR or chemical synthesis.

The present invention also relates to a nucleic acid construct containing the polynucleotide described herein and one or more regulatory sequences operably linked to these sequences. The polynucleotide of the present invention can be manipulated in various ways to ensure the expression of the polypeptide or protein. The nucleic acid construct can be manipulated according to the different expression vectors or requirements before inserting into the vector. Techniques for altering polynucleotide sequences using recombinant DNA methods are known in the art.

In certain embodiments, the nucleic acid construct is a vector. The vector can be a cloning vector, an expression vector, or a homologous recombination vector. The polynucleotide of the present invention can be cloned into many types of vectors, such as plasmids, phagemids, phage derivatives, animal viruses, and cosmids. Cloning vectors can be used to provide the coding sequence of the protein or polypeptide of the present invention. Expression vectors can be provided to cells in the form of bacterial vectors or viral vectors. Expression of the polynucleotide of the present invention is usually achieved by operably linking the polynucleotide of the present invention to a promoter and incorporating the construct into an expression vector. The vector can be suitable for replication and integration into eukaryotic cells. In one or more embodiments, the cloning vector and expression vector are one vector, i.e., a cloning and expression vector. Homologous recombination vectors are used to integrate the expression cassette described herein into the host genome.

A typical expression vector contains expression control sequences that can be used to regulate the expression of a desired nucleic acid sequence, operably linked to the nucleic acid sequence of the present invention or its complementary sequence. The term "expression control sequence" as used herein refers to elements that can be operably linked to a target gene to regulate the transcription, translation, and expression of the target gene, which can be an origin of replication, a promoter of replication, a promoter, a marker gene, or a translation control element, including an enhancer, an operator, a terminator, a ribosome binding site, etc. The choice of expression control sequence depends on the host cell used. In a recombinant expression vector, "operably linked" means that the nucleotide sequence of interest is linked to a regulatory sequence in a way that allows expression of the nucleotide sequence. Those skilled in the art are familiar with methods that can be used to construct expression vectors containing the coding sequence of the fusion protein of the present invention and appropriate transcription/translation control signals. These methods include in vitro recombinant DNA technology, DNA synthesis technology, in vivo recombination technology, etc. The DNA sequence can be effectively linked to an appropriate promoter in the expression vector to direct mRNA synthesis. Representative examples of these promoters are: lac or trp promoters of E. coli; λ phage PL promoter; eukaryotic promoters include CMV immediate early promoter, HSV thymidine kinase promoter, early and late SV40 promoters, retroviral LTRs, and other known promoters that can control gene expression in prokaryotic or eukaryotic cells or their viruses. The expression vector also includes a ribosome binding site for translation initiation and a transcription terminator. In one embodiment, the expression vector can be a commercially available pET28a vector without other special requirements. Illustratively, the nucleotide sequence encoding the optical probe and the expression vector are double-digested with BamHI and EcoRI respectively, and then the digested products of the two are ligated to obtain a recombinant expression vector. The present invention has no special restrictions on the specific steps and parameters of digestion and ligation, and conventional steps and parameters in the art can be used.

After obtaining the recombinant expression vector, the vector is transformed into a host cell to produce a protein or peptide including the fusion protein. This transfer process can be carried out by conventional techniques known to those skilled in the art, such as transformation or transfection. The host cell of the present invention refers to a cell capable of accepting and accommodating a recombinant DNA molecule, which is the site of recombinant gene amplification, and an ideal recipient cell should satisfy the two conditions of easy accessibility and easy proliferation. The "recipient cells" of the present invention may include prokaryotic and eukaryotic cells, specifically bacterial cells, yeast cells, insect cells and mammalian cells. Specifically, they may be bacterial cells such as Escherichia coli, Streptomyces spp. and Salmonella typhimurium, fungal cells such as yeast, plant cells, Drosophila S2 or Sf9 insect cells, animal cells such as CHO, COS, HEK293, HeLa cells, or Bowes melanoma cells including, but not limited to, the aforementioned host cells. Said host cells are preferably various cells that promote gene product expression or fermentation production, and such cells are well known and commonly used in the art. One exemplary host cell used in embodiments of the present invention is the E. coli JM109-DE3 strain. It is well known to those of ordinary skill in the art how to select suitable vectors, promoters, enhancers and host cells.

The methods of transfer to host cells described herein are conventional in the art and include calcium phosphate or calcium chloride co-precipitation, DEAE-mannan-mediated transfection, lipo-transfection, natural susceptibility, chemically mediated transfer or electroporation. When the host is a prokaryote such as E. coli, the method is preferably treatment with CaCl₂ or MgCl₂, and the steps used are well known in the art. When the host cell is a eukaryotic cell, the following DNA transfection methods can be selected: calcium phosphate co-precipitation method, conventional mechanical methods such as microinjection, electroporation, liposome packaging, etc.

In the present invention, after transferring the expression vector into the host cell, the host cell into which the expression vector has been transferred is subjected to expansion expression culture, and the arginine optical probe is isolated. The expansion expression culture of the host cell can be carried out using conventional methods. Depending on the type of host cell used, the medium used in the culture can be various conventional media. Culture is carried out under conditions suitable for the growth of the host cell.

In the present invention, the optical probe is expressed intracellularly, on the cell membrane, or secreted extracellularly. If necessary, the recombinant protein can be separated or purified using various separation methods based on its physical, chemical, and other properties. The present invention has no special restrictions on the method for isolating the arginine fluorescent protein, and conventional methods for isolating fusion proteins in the art can be used. These methods are well known to those skilled in the art and include, but are not limited to: conventional renaturation treatment, salting-out method, centrifugation, osmotic lysis, ultrasonic treatment, ultracentrifugation, molecular sieve chromatography, adsorption chromatography, ion exchange chromatography, high-performance liquid chromatography (HPLC), and various other liquid chromatography techniques and combinations of these methods. In one embodiment, affinity chromatography using a His tag is used for the separation of the optical probe.

The present invention also provides the application of the arginine optical probe in real-time localization, quantitative detection of arginine, and high-throughput compound screening. In one aspect, the arginine optical probe is preferably linked to signal peptides from different parts of the cell, transferred into the cell, and real-time localization of arginine is performed by detecting the intensity of the fluorescent signal in the cell; quantitative detection of the corresponding arginine is performed through the arginine standard titration curve combined with the change of the fluorescent signal. The change in the fluorescent signal is displayed by, for example, the ratio of the normalized fluorescent signal. In embodiments involving cpYFP, the ratio is the ratio of the 485 nm fluorescent signal to the 420 nm fluorescent signal of the sample divided by the corresponding ratio of the control. The arginine standard titration curve of the present invention is drawn according to the fluorescent signal of the arginine optical probe under different concentrations of arginine. The arginine optical probe of the present invention is directly transferred into cells, and during real-time localization and quantitative detection of arginine, time-consuming sample processing steps are not required, making it more accurate. When the arginine optical probe of the present invention is used for high-throughput compound screening, different compounds are added to the cell culture medium, and the change in arginine content is determined to screen out compounds that affect the change in arginine content. The application of the arginine optical probe in real-time localization, quantitative detection of arginine, and high-throughput compound screening in the present invention is for non-diagnostic and therapeutic purposes and does not involve the diagnosis and treatment of diseases.

The present invention also provides a detection kit including the optical probe, nucleic acid molecule, nucleic acid construct, and/or cell described herein. The kit also contains other reagents required for detecting arginine. Such other reagents are well known in the art, such as buffers, cell culture media, arginine standards. An exemplary buffer is, for example, 100 mM HEPES and 100 mM NaCl, pH 7.4.

Partial specific embodiments:
Item 1. An arginine-sensitive polypeptide, which is a variant of an arginine-binding protein, and:
(1) has the sequence shown by SEQ ID NO: 1 and has mutations at 1, 2, 3 or more positions selected from: T32, E37, F71, G89, M90, D91, R96, E135, T138, T139, H140, D177, where the amino acid mutations include amino acid modifications, substitutions or deletions; or
(2) is a sequence that has at least 70% sequence identity with the sequence described in (1), has the mutations described in (1), and retains arginine sensitivity,

Preferably, the mutations are selected from 1, 2, 3 or more of T32A, E37A, F71A, G89A, M90A, D91A, R96A, E135A, T138A, T139A, H140A, D177A.

Item 2. An optical probe, comprising the arginine-sensitive polypeptide according to Item 1 and an optically active polypeptide or a functional variant thereof, where the optically active polypeptide or a functional variant thereof is located within the sequence of the arginine-sensitive polypeptide,
Preferably, the optically active polypeptide is located between residues 200-203 of the arginine-sensitive polypeptide,
More preferably, the optically active polypeptide is located at the 200/203 site of the arginine-sensitive polypeptide.

Item 3. The optical probe according to Item 2, characterized in that the optically active polypeptide is a fluorescent protein or a functional fragment thereof,
Preferably, the fluorescent protein is cpYFP as shown by SEQ ID NO: 3.

Item 4. The optical probe according to Item 2 or 3, characterized in that the optical probe is a probe with cpYFP inserted at the 200/203 site of the arginine-sensitive polypeptide and having one or more mutations selected from T32A, E37A, F71A, G89A, M90A, D91A, R96A, E135A, T138A, T139A, H140A, D177A,

Preferably, the optical probe has the sequence shown by any one of SEQ ID NO: 4-15, or a sequence with at least 70% sequence identity thereto.

Item 5. A nucleic acid molecule, comprising:
(a) the coding sequence of the optical probe according to any one of Items 2-4, or
(b) the complementary sequence of (a), or
(c) a fragment of (a) or (c),

Preferably, the fragment is a primer.

Item 6. A nucleic acid construct comprising the nucleic acid molecule according to Item 5,

Preferably, the nucleic acid construct is a cloning vector, expression vector or recombinant vector.

Item 7. A host cell, which:
(1) expresses the optical probe according to any one of Items 2-4;
(2) comprises the nucleic acid molecule according to Item 5; or
(3) comprises the nucleic acid construct according to Item 6.

Item 8. A method for preparing the optical probe according to any one of Items 2-4, comprising: providing the host cell according to Item 7, culturing the host cell under conditions for expression of the optical probe, and isolating the optical probe or fusion polypeptide.

Item 9. Use of the optical probe according to any one of Items 2-4, the nucleic acid sequence according to Item 5, the nucleic acid construct according to Item 6, or the host cell according to Item 7 in detecting arginine in a sample, screening compounds, or localizing arginine inside/outside cells,

Preferably,
The detection of arginine in a sample comprises the steps of: contacting the optical probe or the host cell with the sample, detecting the optical change of the optically active polypeptide, and quantifying arginine in the sample based on the optical change of the optically active polypeptide,
The screening of compounds comprises the steps of: contacting the optical probe or the host cell with a candidate compound in a system containing arginine, detecting the optical change of the optically active polypeptide, and screening the compound based on the optical change of the optically active polypeptide,
The localization of arginine inside/outside cells comprises the steps of: contacting a system containing arginine with the optical probe or the host cell, and detecting the optical change of the optically active polypeptide.

Item 10. A detection kit, comprising:
(1) the optical probe according to any one of Items 2-4,
(2) the nucleic acid sequence according to Item 5,
(3) the nucleic acid construct according to Item 6, or
(4) the cell according to Item 7, and

Other reagents required for detecting arginine using the optical probe,
Preferably, the detection kit further comprises one or more reagents selected from: buffers, culture media, arginine standards.

In this document, concentrations, contents, percentages, and other numerical values can be expressed in the form of ranges. It should also be understood that the use of such range forms is for convenience and brevity and should be flexibly interpreted as including the numerically specified limits of the range, as well as all individual numerical values or subranges included within the range.

### EXAMPLES

The following examples describe the arginine fluorescent probe provided by the present invention in detail, but they should not be construed as limiting the protection scope of the present invention.

### I. Experimental Materials and Reagents

The examples mainly use conventional genetic engineering molecular biology cloning methods, cell culture, and imaging methods, which are well known to those of ordinary skill in the art, such as: Jan Roskams et al., "Molecular Biology Experimental Reference Manual"; J. Sambrook, D. W. Russell, translated by Huang Peitang et al.: "Molecular Cloning: A Laboratory Manual" (3rd edition, August 2002, published by Science Press, Beijing); Freshney et al., "Animal Cell Culture: A Basic Technical Guide" (5th edition), translated by Zhang Jingbo, Xu Cunsuan et al.; J.S. Bonifacino, M. Dasso et al., "Essential Cell Biology Experimental Guide", translated by Zhang Jingbo et al.

The pET28a-cpYFP and pET28a-arginine-binding protein plasmids used in the examples were constructed by the Protein Laboratory of East China University of Science and Technology, and the pET28a plasmid vector was purchased from Invitrogen. All primers used for PCR were synthesized, purified, and identified as correct by mass spectrometry by Shanghai Jierui Bioengineering Technology Co., Ltd. The expression plasmids constructed in the examples were all sequenced, and the sequencing was completed by Huada Gene Co., Ltd. and Jieli Sequencing Co., Ltd. The Taq DNA polymerase used in each example was purchased from Dongsheng Biology, pfu DNA polymerase was purchased from Tiangen Biochemical Technology (Beijing) Co., Ltd., and primeSTAR DNA polymerase was purchased from TaKaRa Co., Ltd. The three polymerases were provided with corresponding polymerase buffers and dNTP when purchased. Restriction endonucleases such as BamHI, BglII, HindIII, NdeI, XhoI, EcoRI, SpeI, T4 ligase, and T4 phosphorylase (T4 PNK) were purchased from Fermentas Co., Ltd., with corresponding buffers and the like provided when purchased. Transfection reagent Lip2000 Kit was purchased from Invitrogen Co., Ltd. Arginine and the like were purchased from Sigma Co., Ltd. Unless otherwise specified, chemical reagents such as inorganic salts were purchased from sigmaaldrich Co., Ltd. HEPES salt, ampicillin (Amp), and puromycin were purchased from Ameresco Co., Ltd.; 96-well detection black plates and 384-well fluorescence detection black plates were purchased from Grenier Co., Ltd.

The DNA purification kit used in the examples was purchased from BBI Co., Ltd. (Canada), and the ordinary plasmid mini-prep kit was purchased from Tiangen Biochemical Technology (Beijing) Co., Ltd. The cloning strain Mach1 was purchased from Invitrogen Co., Ltd. Nickel column affinity chromatography column and desalting column fillers were from GE healthcare Co., Ltd.

The main instruments used in the examples: Biotek Synergy 2 multifunctional microplate reader (Bio-Tek, USA), X-15R high-speed refrigerated centrifuge (Beckman, USA), Microfuge22R desktop high-speed refrigerated centrifuge (Beckman, USA), PCR amplifier (Biometra, Germany), ultrasonic disruptor (Ningbo Xinzhi Co., Ltd.), nucleic acid electrophoresis apparatus (Shenneng Bocai Co., Ltd.), fluorescence spectrophotometer (Varian, USA), CO₂ constant temperature cell incubator (SANYO), inverted fluorescence microscope (Nikon, Japan).

### II. Molecular Biology Methods and Cell Experimental Methods

### II.1 Polymerase Chain Reaction (PCR):

### 1. PCR for amplification of target fragments:

This method is mainly used for gene fragment amplification and colony PCR identification of positive clones. The PCR amplification reaction system is as follows: 0.5-1 µL of template sequence, 0.5 µL of forward primer (25 µM), 0.5 µL of reverse primer (25 µM), 5 µL of 10×pfu buffer, 0.5 µL of pfu DNA polymerase, 1 µL of dNTP (10 mM), 41.5-42 µL of sterile ultrapure water (ddH2O), with a total volume of 50 µL. The PCR amplification program is as follows: denaturation at 95 °C for 2-10 minutes, 30 cycles (94-96 °C for 30-45 seconds, 50-65 °C for 30-45 seconds, 72 °C for a certain time (600 bp/min)), and extension at 72°C for 10 minutes.

### 2. PCR for amplification of long fragments (>2500 bp):

The long fragment amplification used in the present invention is mainly reverse PCR amplification of vectors, which is used in the following examples as a technique for obtaining site-directed mutations. Reverse PCR primers are designed at the mutation site, and the 5' end of one primer contains the nucleotide sequence of the mutation. The amplified product contains the corresponding mutation site. The long fragment amplification PCR reaction system is as follows: 1 µL of template sequence (10 pg-1 ng), 0.5 µL of forward primer (25 µM), 0.5 µL of reverse primer (25 µM), 10 µL of 5×PrimerSTAR buffer, 0.5 µL of PrimerSTAR DNA polymerase, 4 µL of dNTP (2.5 mM), 33.5 µL of sterile ultrapure water (ddH₂O), with a total volume of 50 µL. The PCR amplification program is as follows: denaturation at 95 °C for 5 minutes, 30 cycles (98 °C for 10 seconds, 50-68 °C for 5-15 seconds, 72 °C for a certain time (1000 bp/min)), and extension at 72 °C for 10 minutes; or denaturation at 95 °C for 5 minutes, 30 cycles (98 °C for 10 seconds, 68 °C for a certain time (1000 bp/min)), and extension at 72 °C for 10 minutes.

### II.2 Nucleic Acid Endonuclease Digestion Reaction:

The system for double digestion of the plasmid vector is as follows: 20 µL of plasmid vector (about 1.5 µg), 5 µL of 10×buffer, 1-2 µL of restriction endonuclease 1, 1-2 µL of restriction endonuclease 2, and sterile ultrapure water to make up to a total volume of 50 µL. The reaction condition is 37 °C for 1-7 hours.

### II.3 DNA Fragment 5' End Phosphorylation Reaction

Plasmids or genomes extracted from microorganisms have phosphate groups at their ends, while PCR products do not, so it is necessary to add phosphate groups to the 5' end bases of the PCR products. Only DNA molecules with phosphate groups at their ends can undergo ligation reactions. The phosphorylation reaction system is as follows: 5-8 µL of PCR product fragment DNA sequence, 1 µL of 10×T4 ligase buffer, 1 µL of T4 polynucleotide kinase (T4 PNK), 0-3 µL of sterile ultrapure water, with a total volume of 10 µL. The reaction condition is 37°C for 30 minutes to 2 hours, then inactivate at 72 °C for 20 minutes.

### II.4 Ligation Reaction of Target Fragment and Vector

The ligation methods between different fragments and vectors are different, and three ligation methods are used in the present invention:

### 1. Blunt-end ligation of short blunt-end fragments and linearized vectors

The principle of this method is that the blunt-end product obtained by PCR is phosphorylated at the 5' end of the DNA fragment under the action of T4 PNK, and then ligated with the linearized vector under the action of PEG4000 and T4 DNA ligase to obtain a recombinant plasmid. The homologous recombination ligation system is as follows: 4 µL of T4 PNK-treated DNA fragment, 4 µL of linearized vector fragment, 1 µL of PEG4000, 1 µL of 10×T4 ligase buffer, 1 µL of T4 DNA ligase, with a total volume of 10 µL. The reaction condition is 22 °C for 30 minutes.

### 2. Ligation of DNA fragments with sticky ends and vector fragments with sticky ends

DNA fragments cut by restriction endonucleases usually produce protruding sticky ends, so they can be ligated with vector fragments with complementary sticky ends to form recombinant plasmids. The ligation reaction system is as follows: 1-7 µL of digested PCR product fragment DNA, 0.5-7 µL of digested plasmid, 1 µL of 10×T4 ligase buffer, 1 µL of T4 DNA ligase, and sterile ultrapure water to make up to a total volume of 10 µL. The reaction condition is 16 °C for 4-8 hours.

### 3. Self-circularization ligation reaction of 5' end phosphorylated DNA fragment products after introducing site-directed mutations by reverse PCR

The 3' end and 5' end of the linearized vector are ligated by self-circularization ligation reaction of 5' end phosphorylated DNA fragments to obtain recombinant plasmids. The self-circularization ligation reaction system is as follows: 10 µL of phosphorylation reaction system, 0.5 µL of T4 ligase (5 U/µL), with a total volume of 10.5 µL. The reaction condition is 16 °C for 4-16 hours.

### II.5 Preparation and Transformation of Competent Cells

Preparation of competent cells:
1. Pick a single colony (such as Mach1) and inoculate it into 5 mL of LB medium, and incubate overnight in a shaker at 37 °C.
2. Transfer 0.5-1 mL of the overnight culture to 50 mL of LB medium, and culture at 37 °C with 220 rpm for 3 to 5 hours until the OD600 reaches 0.5.
3. Pre-cool the cells on ice for 2 hours.
4. Centrifuge at 4000 rpm for 10 minutes at 4 °C.
5. Discard the supernatant, resuspend the cell pellet with 5 mL of pre-cooled buffer, and after homogenization, add resuspension buffer to a final volume of 50 mL.
6. Ice bath for 45 minutes.
7. Centrifuge at 4000 rpm for 10 minutes at 4 °C, and resuspend the bacteria with 5 mL of ice-pre-cooled storage buffer.
8. Place 100 µL of bacterial solution in each EP tube, and store at -80 °C or in liquid nitrogen.

Resuspension buffer: CaCl₂ (100 mM), MgCl₂ (70 mM), NaAc (40 mM)
Storage buffer: 0.5 mL DMSO, 1.9 mL 80% glycerol, 1 mL 10×CaCl₂ (1 M), 1 mL 10×MgCl₂ (700 mM), 1 mL 10×NaAc (400 mM), 4.6 mL ddH₂O

Transformation of competent cells:
1. Take 100 µL of competent cells and thaw on ice.
2. Add an appropriate volume of ligation product, gently pipette to mix, and ice bath for 30 minutes. Usually, the volume of the added ligation product is less than 1/10 of the volume of the competent cells.
3. Heat-shock the bacterial solution in a 42°C water bath for 90 seconds, and quickly transfer to an ice bath for 5 minutes.
4. Add 500 µL of LB, and culture at 37°C with 200 rpm for 1 hour in a constant temperature shaker.
5. Centrifuge the bacterial solution at 4000 rpm for 3 minutes, leave 200 µL of supernatant to resuspend the bacterial cells, evenly spread on the surface of the agar plate containing appropriate antibiotics, and invert the plate in a 37°C constant temperature incubator overnight.

### II.6 Protein Expression, Purification and Fluorescence Detection

1. Transform the pET28a-based arginine probe plasmid into BL21(DE3), culture overnight in an inverted manner, pick a clone from the plate into a 250 ml conical flask, place in a 37 °C shaker, culture at 220 rpm until OD = 0.4-0.8, add IPTG (1 M) at 1/1000 (v/v), and induce expression at 18 °C for 24-36 h.
2. After induction expression, collect the bacteria by centrifugation at 4000 rpm for 30 minutes, add 50 mM phosphate buffer to resuspend the bacterial pellet, and ultrasonically disrupt until the bacteria are clarified. Centrifuge at 9600 rpm at 4 °C for 20 minutes.
3. The centrifuged supernatant is purified by a self-assembled nickel column affinity chromatography column to obtain the protein, and the protein after nickel column affinity chromatography is then passed through a self-assembled desalting column to obtain the protein dissolved in 20 mM MOPS buffer (pH 7.4) or phosphate buffer PBS.
4. The purified arginine-binding protein mutant protein is identified by SDS-PAGE, and the probe is diluted with assay buffer (100 mM HEPES, 100 mM NaCl, pH 7.3) or phosphate buffer PBS to a final concentration of 5-10 µM protein solution. Prepare arginine into a stock solution with a final concentration of 1 M using assay buffer (20 mM MOPS, pH 7.4) or phosphate buffer PBS.
5. Take 100 µl of 5 µM protein solution, incubate at 37 °C for 5 min, add arginine respectively and mix to a final concentration of 100 mM, and measure the light absorption of the protein at 340 nm using a multifunctional fluorescence microplate reader.
6. Take 100 µl of 1 µM fluorescent probe solution, incubate at 37 °C for 5 min, add arginine for titration, and measure the fluorescence intensity of the protein at 528 nm emission after 485 nm fluorescence excitation. The measurement of fluorescence excitation and emission of samples is completed using a multifunctional fluorescence microplate reader.
7. Take 100 µl of 1 µM fluorescent probe solution, incubate at 37 °C for 5 min, add arginine, and measure the absorption spectrum and fluorescence spectrum of the probe protein. The measurement of absorption spectrum and fluorescence spectrum of samples is completed by a spectrophotometer and a fluorescence spectrophotometer.

### II.7 Transfection and Fluorescence Detection of Mammalian Cells

1. Transfect the PAAV-based arginine probe plasmid into HEK293 using transfection reagent Lipofectamine2000 (Invitrogen), and culture in a cell incubator at 37 °C with 5% CO₂. Perform fluorescence detection after sufficient expression of the foreign gene for 24-36 h.
2. After induction expression, rinse the adherent HEK293 cells three times with PBS, and place them in HBSS solution for fluorescence microscope and microplate reader detection respectively.

### Example 1: Arginine-Binding Protein Plasmid

Amplify the STM4351 gene from the Agrobacterium tumefaciens gene by PCR, recover the PCR product after gel electrophoresis, digest with BamHI and EcoRI, and perform the same double digestion on the pET28a vector. Ligate with T4 DNA ligase, transform the ligation product into Trans5a, spread the transformed Trans5a on an LB plate (kanamycin 100 µg/mL), and culture at 37 °C overnight. Extract plasmids from the growing Trans5a transformants and perform PCR identification. After the positive plasmids are sequenced correctly, proceed with subsequent plasmid construction.

### Example 2: Expression and Detection of cpYFP Optical Probe Binding Site Mutations at the 200/203 Insertion Site

In this example, based on pET28a-STM4351, the following sites were selected for inserting cpYFP to obtain corresponding plasmids: pET28a-STM4351-200/203-cpYFP. Mutations were made on the basis of pET28a-STM4351-200/203-cpYFP to obtain plasmids with the following mutations: T32A, E37A, F71A, G89A, M90A, D91A, R96A, E135A, T138A, T139A, H140A, D177A. The sequences of exemplary optical probes are shown in Table 1.

**Table 1, Sequences of Optical Probes**

| **Sequence** | **Mutation Site** |
|---|---|
| SEQ ID NO:4 | T32A |
| SEQ ID NO:5 | E37A |
| SEQ ID NO:6 | F71A |
| SEQ ID NO:7 | G89A |
| SEQ ID NO:8 | M90A |
| SEQ ID NO:9 | D91A |
| SEQ ID NO:10 | R96A |
| SEQ ID NO:11 | E135A |
| SEQ ID NO:12 | T138A |
| SEQ ID NO:13 | T139A |
| SEQ ID NO:14 | H140A |
| SEQ ID NO:15 | D177A |

Generate the DNA fragment of cpYFP by PCR, perform 5' end phosphorylation on the DNA fragment and inactivate it, and simultaneously amplify the pET28a-arginine-binding protein linearized vector with different break sites by reverse PCR. Ligate the linearized pET28a-STM4351 and 5' end phosphorylated cpYFP fragment under the action of PEG4000 and T4 DNA ligase to generate recombinant plasmids. Place these plates in a Kodak multifunctional in vivo imaging system, pick clones with yellow fluorescence under FITC channel excitation, and sequencing was completed by Beijing Liuhe Huada Gene Technology Co., Ltd. Shanghai Branch.

Linearize the plasmid pET28a-STM4351-200/203-cpYFP by reverse PCR, where the primers contain the base sequence of the desired mutation site. Phosphorylate and ligate the obtained PCR product under the action of PNK, T4 DNA ligase and PEG4000 to obtain site-directed mutant plasmids at 12 sites: T32A, E37A, F71A, G89A, M90A, D91A, R96A, E135A, T138A, T139A, H140A, D177A, and sequencing was completed by Beijing Liuhe Huada Gene Technology Co., Ltd. Shanghai Branch.

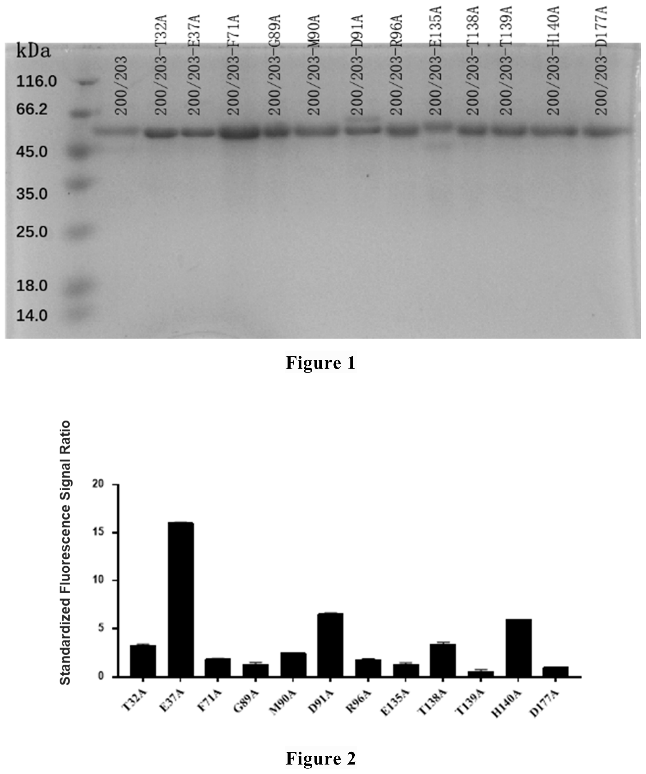

After correct sequencing, transform the recombinant plasmid into BL21(DE3) for induced expression, and purify the protein. The size is around 55 KDa by SDS-PAGE electrophoresis. This size is consistent with the size of the STM4351-cpYFP fusion protein containing a His-tag purification tag expressed by pET28a-STM4351-cpYFP. The results are shown in Figure 1.

Screen the purified STM4351-cpYFP fusion protein for arginine response, and divide the detection signal of the fusion fluorescent protein containing 100 mM arginine by the detection signal of the fusion fluorescent protein without arginine. The results are shown in Figure 2. The detection results show that T32A, E37A, D91A, T138A, and H140A have a response to arginine exceeding 3 times, among which E37A has a response to arginine exceeding 15 times.

Example 3: Titration Curves of cpYFP Optical Probe Binding Site Mutant Probes at the 200/203 Insertion Site

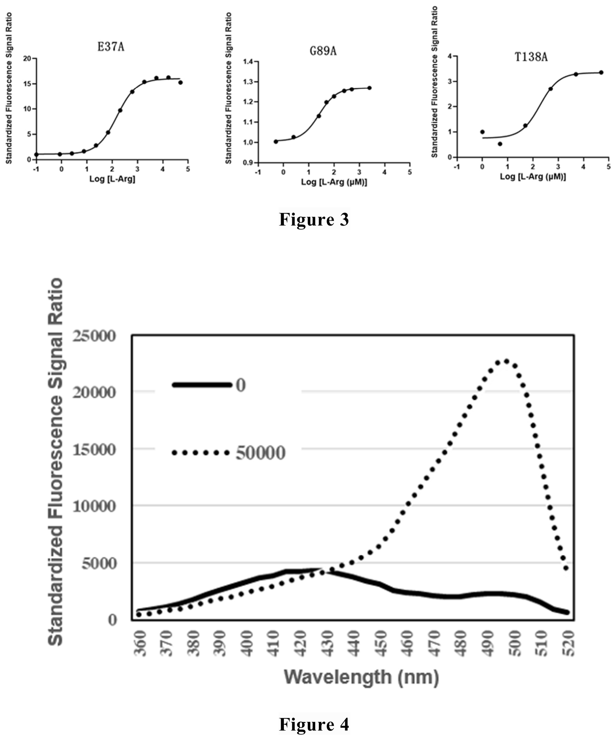

Express and detect the E37A, G89A, and T138A probes according to the method in Example 2. The results are shown in Figure 3. The fluorescence detection results show that the E37A probe has a response to arginine of about 15 times, reaches saturation at 10 mM arginine substrate, and has an affinity for the substrate of 170-210 µM; the G89A and T138A probes have a smaller response to arginine, with response multiples of about 1.29 and 3.14, respectively, and their affinities for the substrate are 23.57 µM and 189.8 µM, respectively.

### Example 4: Spectral Properties of Probe E37A

Purify the mutant STM4351-200/203-E37A-cpYFP with high response multiple and good specificity. After treating the purified arginine fluorescent probe with 0 mM and 100 mM arginine for 10 min respectively, use a fluorescence spectrophotometer to detect the fluorescence spectrum. Determination of emission spectrum: fix the excitation wavelengths at 420 nm and 485 nm respectively, record the emission spectrum at 360-540 nm, and read once every 5 nm. The spectral curve of the arginine fluorescent probe STM4351-200/203-E37A-cpYFP is shown in Figure 4. After adding 500 mM arginine to the probe, the fluorescence intensity under 420 nm excitation is basically unchanged compared with that of adding 0 mM arginine; the fluorescence intensity under 485 nm excitation increases to about 15.0 times that of adding 0 mM arginine.

### Example 5: Specificity Determination of Arginine Probes

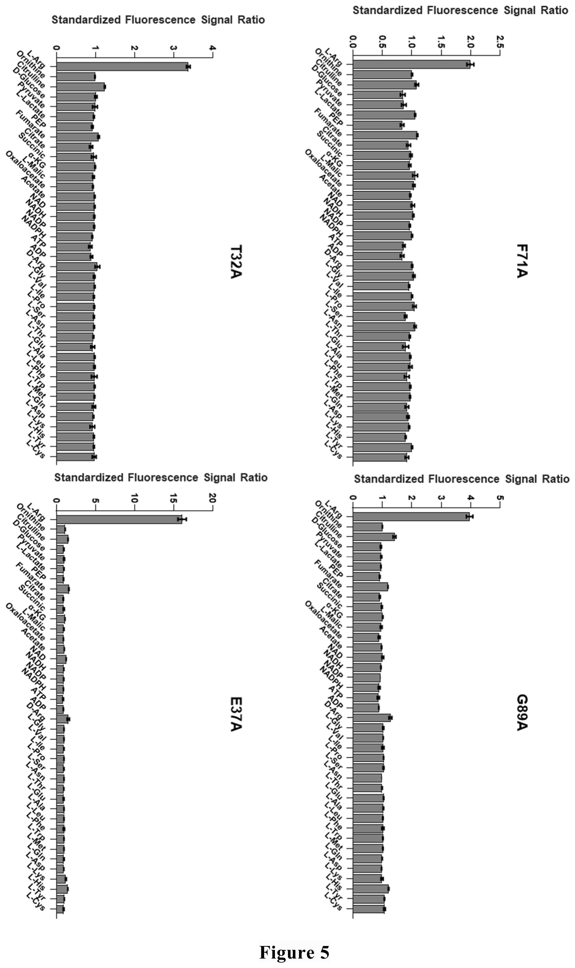

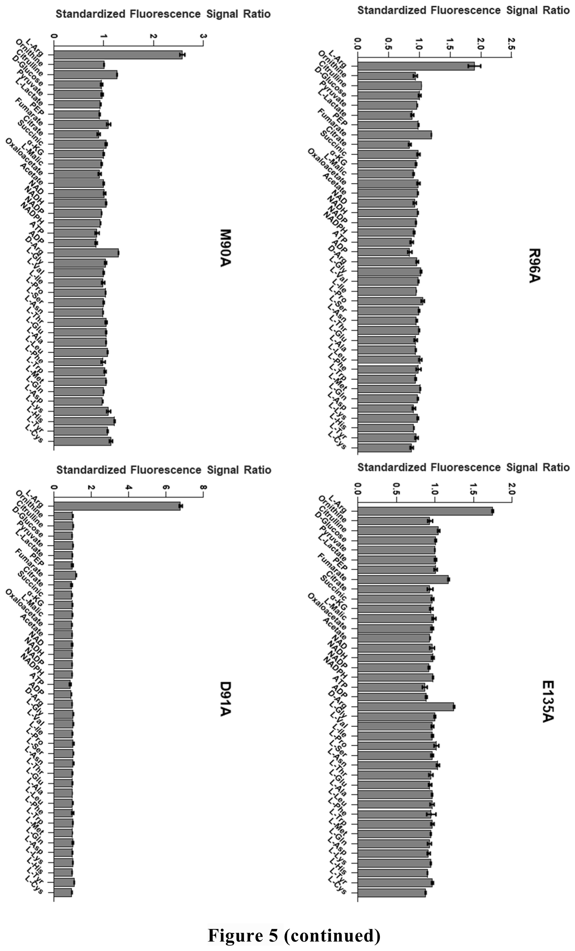

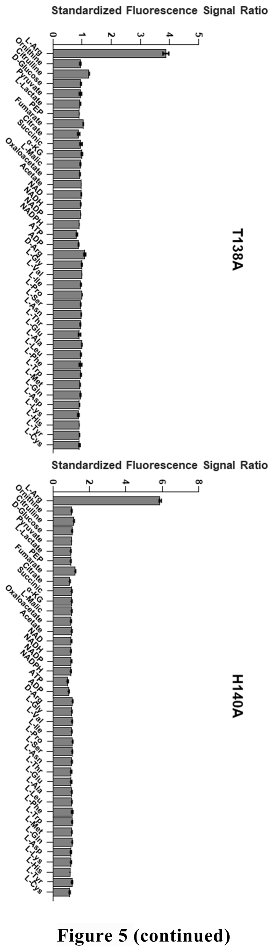

Detect the specificity of the mutants T32A, E37A, F71A, G89A, M90A, D91A, R96A, E135A, T138A, and H140A in Figure 2. The results show that the mutant probes have good specificity for arginine, as shown in Figure 5.

### Example 6: Affinity of E37A Probe for Substrate under Different pH Values

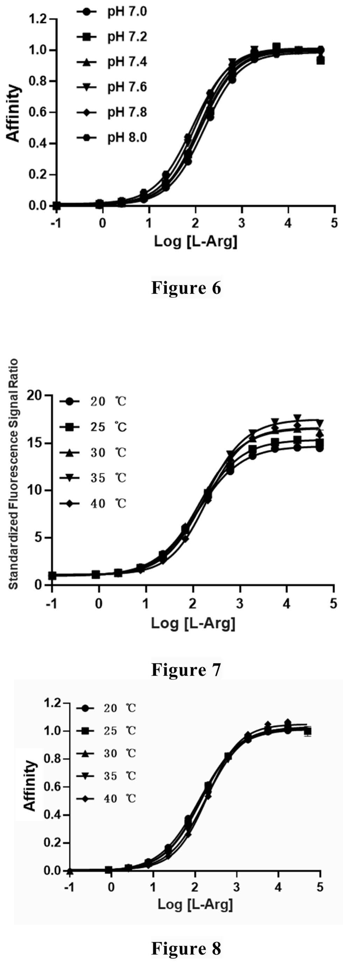

Purify STM4351-200/203-E37A-cpYFP as in Example 2, and test its binding ability to the substrate arginine under different pH conditions. The results show that the affinity for the substrate is basically unchanged under different pH conditions, as shown in Figure 6.

### Example 7: Temperature Sensitivity of E37A Probe

Purify STM4351-200/203-E37A-cpYFP as in Example 2, and titrate its binding ability to the substrate arginine under different temperature conditions. The results show that the probe has the best binding response to the substrate at 35 °C, and the lowest response multiple at 20 °C, as shown in Figure 7.

### Example 8: Affinity of E37A Probe for Substrate at Different Temperatures

Purify STM4351-200/203-E37A-cpYFP as in Example 2, and test its binding ability to the substrate arginine at different temperatures. The results show that the affinity for the substrate is basically unchanged at different temperatures, as shown in Figure 8.

### Example 9: Subcellular Organelle Localization of Probes and Their Performance in Subcellular Organelles

In this example, we fused different localization signal peptides with the C-terminus or N-terminus of the arginine fluorescent probe STM4351-200/203-E37A-cpYFP, and localized the arginine fluorescent probe STM4351-200/203-E37A-cpYFP to different organelles.

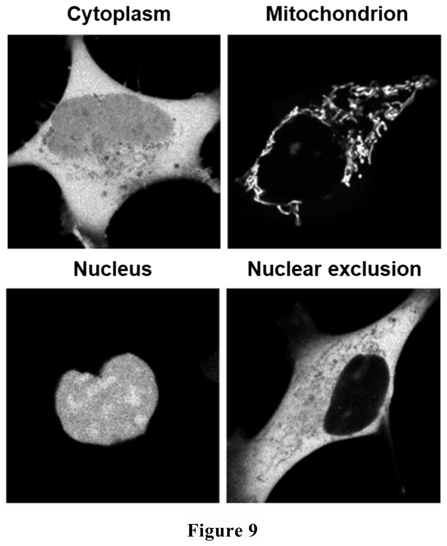

Transfect the plasmid of the arginine fluorescent probe STM4351-200/203-E37A-cpYFP gene fused with different localization signal peptides into HEK293 cells for 36 hours, rinse with PBS, and use an inverted fluorescence microscope to detect fluorescence under the FITC channel in HBSS solution. We found that the arginine fluorescent probe FLIPpro can be localized to subcellular organelles including cytoplasm, mitochondria, nucleus, nuclear exclusion, etc. by fusing with different specific localization signal peptides. The results are shown in Figure 9. Fluorescence is shown in different subcellular structures, and the distribution and intensity of fluorescence are different.

### Example 10: Dynamic Monitoring of Arginine Transmembrane Transport

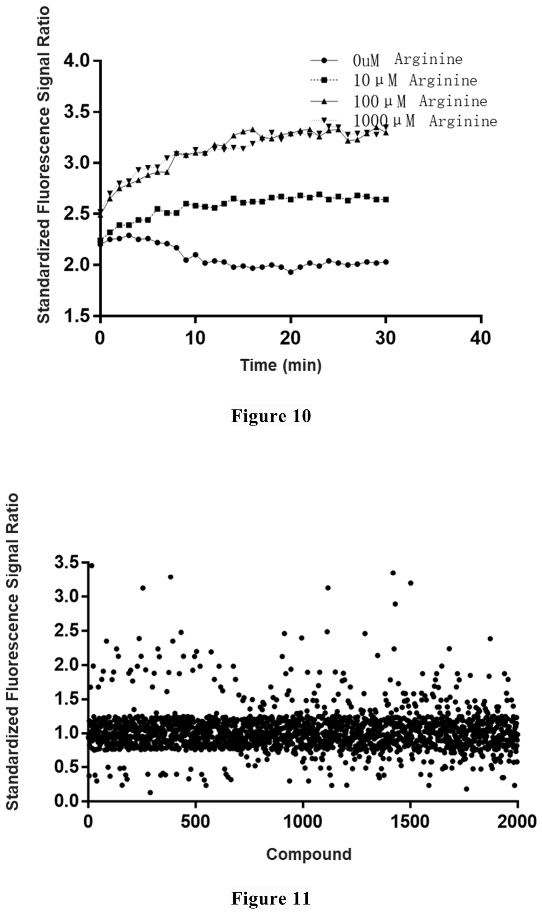

Transfect the plasmid of the cytoplasmically expressed STM4351-200/203-E37A-cpYFP gene into HEK293 cells for 36 hours, rinse with PBS, and detect the change in the ratio of fluorescence intensity at 528 nm emission excited at 420 nm and 485 nm within 40 minutes in HBSS solution. The results are shown in Figure 10. After starvation for 2 hours, 10 µM, 100 µM, and 1000 µM arginine were added respectively and detected for 30 minutes. The ratio 485/420 gradually increased, reaching a maximum of 1.65 times.

### Example 11: High-Throughput Compound Screening at the Live Cell Level

In this example, we performed high-throughput compound screening using HEK293 cells expressing the cytoplasmic arginine probe STM4351-200/203-E37A-cpYFP.

HEK293 cells transfected with the STM4351-200/203-E37A-cpYFP gene were rinsed with PBS, treated in HBSS solution (without arginine) for 1 hour, and then treated with 10 µM compounds for 1 hour. Arginine was added respectively. A microplate reader was used to record the change in the ratio of fluorescence intensity at 528 nm emission excited at 420 nm and 485 nm. Samples not treated with any compound were used as the standard. The results are shown in Figure 11. We found that among cells treated with 2000 compounds, most compounds had little effect on arginine entry into cells. There were 16 compounds that could enhance cellular uptake of arginine, and 11 compounds that could significantly reduce cellular uptake of arginine.

### Example 12: Quantitative Detection of Arginine in Blood and Urine by Probes

In this example, the purified arginine fluorescent probe STM4351-200/203-E37A-cpYFP protein was used to analyze arginine in human blood supernatant and urine.

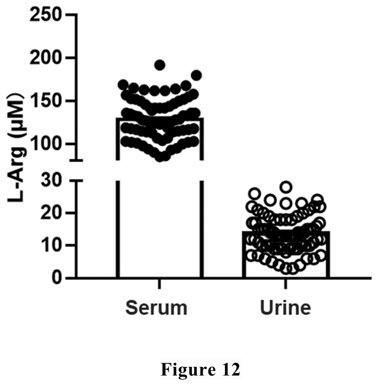

Mix the arginine fluorescent probe STM4351-200/203-E37A-cpYFP fluorescent protein with the diluted blood supernatant and treat for 10 min, then use a microplate reader to detect the ratio of fluorescence intensity at 528 nm emission excited at 420 nm and 485 nm. The urine test is the same as the serum test. The results are shown in Figure 12. It was found that the arginine content in human blood is about 150 µM, and the arginine content in human urine is about 13.7 µM.

The above partial examples only select probes with the E37A mutation as examples. Other mutant probes T32A, E37A, G89A, D91A, T138A, and H140A also show results corresponding to the arginine response results. It can be seen from the above examples that the arginine fluorescent probe provided by the present invention has a relatively small molecular weight, is easy to mature, has large fluorescence dynamic changes and good specificity, and can be expressed in cells through genetic manipulation methods, enabling real-time localization and quantitative detection of arginine inside and outside cells; and can perform high-throughput compound screening.

The above are only preferred embodiments of the present invention. It should be pointed out that for those of ordinary skill in the art, without departing from the principle of the present invention, several improvements and modifications can be made, and these improvements and modifications should also be regarded as the protection scope of the present invention.

### SEQUENCES OF THE APPLICATION

1<STM4351 
ASVSARTLHFGTSATYAPYEFVDADNKIVGFDIDVANAVCKEMQAECSFTNQSFDSLIPSLRFKKFDAVIAGMDMTPKREQQVSFSQPYYEGLSAVV
VTRKGAYHTFADLKGKKVGLENGTTHQRYLQDKQQAITPVAYDSYLNAFTDLKNNRLEGVFGDVAAIGKWLKNNPDYAIMDERASDPDYYGKGL
GIAVRKDNDALLQEINAALDKVKASPEYAQMQEKWFTQ
2<STM4351-200/203-cpYFP protein sequence 
ASVSARTLHFGTSATYAPYEFVDADNKIVGFDIDVANAVCKEMQAECSFTNQSFDSLIPSLRFKKFDAVIAGMDMTPKREQQVSFSQPYYEGLSAVV
VTRKGAYHTFADLKGKKVGLENGTTHQRYLQDKQQAITPVAYDSYLNAFTDLKNNRLEGVFGDVAAIGKWLKNNPDYAIMDERASDYNSDNVYI
MADKQKNGIKANFKIRHNVEDGSVQLADHYQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGYGLKCFARYPDHMKQHDFFKSAMPEGYVQER
TIFFKDDGNYKTRAEVKFEGDTLVNRIELKGIGFKEDGNILGHKLEYNYYGKGLGIAVRKDNDALLQEINAALDKVKASPEYAQMQEKWFTQ
3<cpYFP 
YNSDNVYIMADKQKNGIKANFKIRHNVEDGSVQLADHYQQNTPIGDGPVLLPDNHYLSFQSVLSKDPNEKRDHMVLLEFVTAAGITLGMDELYNV
DGGSGGTGSKGEELFTGVVPILVELDGDVNGHKFSVSGEGEGDATYGKLTLKLICTTGKLPVPWPTLVTTLGYGLKCFARYPDHMKQHDFFKSAMP
EGYVQERTIFFKDDGNYKTRAEVKFEGDTLVNRIELKGIDFKEDGNILGHKLEYN
4<STM4351-200/203-T32A-cpYFP protein sequence 
ASVSARTLHFGTSAAYAPYEFVDADNKIVGFDIDVANAVCKEMQAECSFTNQSFDSLIPSLRFKKFDAVIAGMDMTPKREQQVSFSQPYYEGLSAV
VVTRKGAYHTFADLKGKKVGLENGTTHQRYLQDKQQAITPVAYDSYLNAFTDLKNNRLEGVFGDVAAIGKWLKNNPDYAIMDERASDYNSDNVYI
MADKQKNGIKANFKIRHNVEDGSVQLADHYQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGYGLKCFARYPDHMKQHDFFKSAMPEGYVQER
TIFFKDDGNYKTRAEVKFEGDTLVNRIELKGIGFKEDGNILGHKLEYNYYGKGLGIAVRKDNDALLQEINAALDKVKASPEYAQMQEKWFTQ
5<STM4351-200/203-E37A-cpYFP protein sequence 
ASVSARTLHFGTSATYAPYAFVDADNKIVGFDIDVANAVCKEMQAECSFTNQSFDSLIPSLRFKKFDAVIAGMDMTPKREQQVSFSQPYYEGLSAV

VVTRKGAYHTFADLKGKKVGLENGTTHQRYLQDKQQAITPVAYDSYLNAFTDLKNNRLEGVFGDVAAIGKWLKNNPDYAIMDERASDYNSDNVYI

MADKQKNGIKANFKIRHNVEDGSVQLADHYQQNTPIGDGPVLLPDNHYLSFQSVLSKDPNEKRDHMVLLEFVTAAGITLGMDELYNVDGGSGGT

GSKGEELFTGVVPILVELDGDVNGHKFSVSGEGEGDATYGKLTLKLICTTGKLPVPWPTLVTTLGYGLKCFARYPDHMKQHDFFKSAMPEGYVQER

TIFFKDDGNYKTRAEVKFEGDTLVNRIELKGIGFKEDGNILGHKLEYNYYGKGLGIAVRKDNDALLQEINAALDKVKASPEYAQMQEKWFTQ
6<STM4351-200/203-F71A-cpYFP protein sequence 
ASVSARTLHFGTSATYAPYEFVDADNKIVGFDIDVANAVCKEMQAECSFTNQSADSLIPSLRFKKFDAVIAGMDMTPKREQQVSFSQPYYEGLSAV
VVTRKGAYHTFADLKGKKVGLENGTTHQRYLQDKQQAITPVAYDSYLNAFTDLKNNRLEGVFGDVAAIGKWLKNNPDYAIMDERASDYNSDNVYI
 
MADKQKNGIKANFKIRHNVEDGSVQLADHYQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGYGLKCFARYPDHMKQHDFFKSAMPEGYVQER
TIFFKDDGNYKTRAEVKFEGDTLVNRIELKGIGFKEDGNILGHKLEYNYYGKGLGIAVRKDNDALLQEINAALDKVKASPEYAQMQEKWFTQ
7<STM4351-200/203-G89A-cpYFP protein sequence 
ASVSARTLHFGTSATYAPYEFVDADNKIVGFDIDVANAVCKEMQAECSFTNQSFDSLIPSLRFKKFDAVIAAMDMTPKREQQVSFSQPYYEGLSAVV
VTRKGAYHTFADLKGKKVGLENGTTHQRYLQDKQQAITPVAYDSYLNAFTDLKNNRLEGVFGDVAAIGKWLKNNPDYAIMDERASDYNSDNVYI
MADKQKNGIKANFKIRHNVEDGSVQLADHYQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGYGLKCFARYPDHMKQHDFFKSAMPEGYVQER
TIFFKDDGNYKTRAEVKFEGDTLVNRIELKGIGFKEDGNILGHKLEYNYYGKGLGIAVRKDNDALLQEINAALDKVKASPEYAQMQEKWFTQ
8<STM4351-200/203-M90A -cpYFP protein sequence 
ASVSARTLHFGTSATYAPYEFVDADNKIVGFDIDVANAVCKEMQAECSFTNQSFDSLIPSLRFKKFDAVIAGADMTPKREQQVSFSQPYYEGLSAVV
VTRKGAYHTFADLKGKKVGLENGTTHQRYLQDKQQAITPVAYDSYLNAFTDLKNNRLEGVFGDVAAIGKWLKNNPDYAIMDERASDYNSDNVYI
MADKQKNGIKANFKIRHNVEDGSVQLADHYQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGYGLKCFARYPDHMKQHDFFKSAMPEGYVQER
TIFFKDDGNYKTRAEVKFEGDTLVNRIELKGIGFKEDGNILGHKLEYNYYGKGLGIAVRKDNDALLQEINAALDKVKASPEYAQMQEKWFTQ
9<STM4351-200/203-D91A -cpYFP protein sequence 
ASVSARTLHFGTSATYAPYEFVDADNKIVGFDIDVANAVCKEMQAECSFTNQSFDSLIPSLRFKKFDAVIAGMAMTPKREQQVSFSQPYYEGLSAVV
VTRKGAYHTFADLKGKKVGLENGTTHQRYLQDKQQAITPVAYDSYLNAFTDLKNNRLEGVFGDVAAIGKWLKNNPDYAIMDERASDYNSDNVYI
MADKQKNGIKANFKIRHNVEDGSVQLADHYQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGYGLKCFARYPDHMKQHDFFKSAMPEGYVQER
TIFFKDDGNYKTRAEVKFEGDTLVNRIELKGIGFKEDGNILGHKLEYNYYGKGLGIAVRKDNDALLQEINAALDKVKASPEYAQMQEKWFTQ
10<STM4351-200/203-R96A-cpYFP protein sequence 
ASVSARTLHFGTSATYAPYEFVDADNKIVGFDIDVANAVCKEMQAECSFTNQSFDSLIPSLRFKKFDAVIAGMDMTPKAEQQVSFSQPYYEGLSAV
VVTRKGAYHTFADLKGKKVGLENGTTHQRYLQDKQQAITPVAYDSYLNAFTDLKNNRLEGVFGDVAAIGKWLKNNPDYAIMDERASDYNSDNVYI
MADKQKNGIKANFKIRHNVEDGSVQLADHYQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGYGLKCFARYPDHMKQHDFFKSAMPEGYVQER
TIFFKDDGNYKTRAEVKFEGDTLVNRIELKGIGFKEDGNILGHKLEYNYYGKGLGIAVRKDNDALLQEINAALDKVKASPEYAQMQEKWFTQ
11<STM4351-200/203-E135A-cpYFP protein sequence 
ASVSARTLHFGTSATYAPYEFVDADNKIVGFDIDVANAVCKEMQAECSFTNQSFDSLIPSLRFKKFDAVIAGMDMTPKREQQVSFSQPYYEGLSAVV
VTRKGAYHTFADLKGKKVGLANGTTHQRYLQDKQQAITPVAYDSYLNAFTDLKNNRLEGVFGDVAAIGKWLKNNPDYAIMDERASDYNSDNVYI
MADKQKNGIKANFKIRHNVEDGSVQLADHYQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGYGLKCFARYPDHMKQHDFFKSAMPEGYVQER
TIFFKDDGNYKTRAEVKFEGDTLVNRIELKGIGFKEDGNILGHKLEYNYYGKGLGIAVRKDNDALLQEINAALDKVKASPEYAQMQEKWFTQ
12<STM4351-200/203-T138A -cpYFP protein sequence 
ASVSARTLHFGTSATYAPYEFVDADNKIVGFDIDVANAVCKEMQAECSFTNQSFDSLIPSLRFKKFDAVIAGMDMTPKREQQVSFSQPYYEGLSAVV
VTRKGAYHTFADLKGKKVGLENGATHQRYLQDKQQAITPVAYDSYLNAFTDLKNNRLEGVFGDVAAIGKWLKNNPDYAIMDERASDYNSDNVYI
MADKQKNGIKANFKIRHNVEDGSVQLADHYQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGYGLKCFARYPDHMKQHDFFKSAMPEGYVQER
TIFFKDDGNYKTRAEVKFEGDTLVNRIELKGIGFKEDGNILGHKLEYNYYGKGLGIAVRKDNDALLQEINAALDKVKASPEYAQMQEKWFTQ
13<STM4351-200/203-T139A-cpYFP protein sequence 
ASVSARTLHFGTSATYAPYEFVDADNKIVGFDIDVANAVCKEMQAECSFTNQSFDSLIPSLRFKKFDAVIAGMDMTPKREQQVSFSQPYYEGLSAVV
VTRKGAYHTFADLKGKKVGLENGTAHQRYLQDKQQAITPVAYDSYLNAFTDLKNNRLEGVFGDVAAIGKWLKNNPDYAIMDERASDYNSDNVYI
MADKQKNGIKANFKIRHNVEDGSVQLADHYQQNTPIGDGPVLLPDNHYLSFQSVLSKDPNEKRDHMVLLEFVTAAGITLGMDELYNVDGGSGGT
 
GSKGEELFTGVVPILVELDGDVNGHKFSVSGEGEGDATYGKLTLKLICTTGKLPVPWPTLVTTLGYGLKCFARYPDHMKQHDFFKSAMPEGYVQER
TIFFKDDGNYKTRAEVKFEGDTLVNRIELKGIGFKEDGNILGHKLEYNYYGKGLGIAVRKDNDALLQEINAALDKVKASPEYAQMQEKWFTQ
14<STM4351-200/203-H140A-cpYFP protein sequence 
ASVSARTLHFGTSATYAPYEFVDADNKIVGFDIDVANAVCKEMQAECSFTNQSFDSLIPSLRFKKFDAVIAGMDMTPKREQQVSFSQPYYEGLSAVV
VTRKGAYHTFADLKGKKVGLENGTTAQRYLQDKQQAITPVAYDSYLNAFTDLKNNRLEGVFGDVAAIGKWLKNNPDYAIMDERASDYNSDNVYI
MADKQKNGIKANFKIRHNVEDGSVQLADHYQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGYGLKCFARYPDHMKQHDFFKSAMPEGYVQER
TIFFKDDGNYKTRAEVKFEGDTLVNRIELKGIGFKEDGNILGHKLEYNYYGKGLGIAVRKDNDALLQEINAALDKVKASPEYAQMQEKWFTQ
15<STM4351-200/203-D177A-cpYFP protein sequence

ASVSARTLHFGTSATYAPYEFVDADNKIVGFDIDVANAVCKEMQAECSFTNQSFDSLIPSLRFKKFDAVIAGMDMTPKREQQVSFSQPYYEGLSAVV
VTRKGAYHTFADLKGKKVGLENGTTHQRYLQDKQQAITPVAYDSYLNAFTDLKNNRLEGVFGAVAAIGKWLKNNPDYAIMDERASDYNSDNVYI
MADKQKNGIKANFKIRHNVEDGSVQLADHYQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGYGLKCFARYPDHMKQHDFFKSAMPEGYVQER
TIFFKDDGNYKTRAEVKFEGDTLVNRIELKGIGFKEDGNILGHKLEYNYYGKGLGIAVRKDNDALLQEINAALDKVKASPEYAQMQEKWFTQ

## Claims

1. An arginine-sensitive polypeptide, which is a variant of an arginine-binding protein, and:
(1) has the sequence shown by SEQ ID NO: 1 and has mutations at 1, 2, 3 or more positions selected from: T32, E37, F71, G89, M90, D91, R96, E135, T138, T139, H140, D177, where the amino acid mutations include amino acid modifications, substitutions or deletions; or
(2) is a sequence that has at least 70% sequence identity with the sequence described in (1), has the mutations described in (1), and retains arginine sensitivity,
preferably, the mutations are selected from 1, 2, 3 or more of T32A, E37A, F71A, G89A, M90A, D91A, R96A, E135A, T138A, T139A, H140A, D177A.

2. An optical probe, comprising the arginine-sensitive polypeptide according to claim 1 and an optically active polypeptide or a functional variant thereof, where the optically active polypeptide or a functional variant thereof is located within the sequence of the arginine-sensitive polypeptide,
preferably, the optically active polypeptide is located between residues 200-203 of the arginine-sensitive polypeptide,
more preferably, the optically active polypeptide is located at the 200/203 site of the arginine-sensitive polypeptide.

3. The optical probe according to claim 2, **characterized in that** the optically active polypeptide is a fluorescent protein or a functional fragment thereof,
preferably, the fluorescent protein is cpYFP as shown by SEQ ID NO: 3.

4. The optical probe according to claim 2 or 3, **characterized in that** the optical probe is a probe with cpYFP inserted at the 200/203 site of the arginine-sensitive polypeptide and having one or more mutations selected from T32A, E37A, F71A, G89A, M90A, D91A, R96A, E135A, T138A, T139A, H140A, D177A,
preferably, the optical probe has the sequence shown by any one of SEQ ID NO: 4-15, or a sequence with at least 70% sequence identity thereto.

5. A nucleic acid molecule, comprising:
(a) the coding sequence of the optical probe according to any one of claims 2-4, or
(b) the complementary sequence of (a), or
(c) a fragment of (a) or (c),
preferably, the fragment is a primer.

6. A nucleic acid construct comprising the nucleic acid molecule according to claim 5,
preferably, the nucleic acid construct is a cloning vector, expression vector or recombinant vector.

7. A host cell, which:
(1) expresses the optical probe according to any one of claims 2-4;
(2) comprises the nucleic acid molecule according to claim 5; or
(3) comprises the nucleic acid construct according to claim 6.

8. A method for preparing the optical probe according to any one of claims 2-4, comprising: providing the host cell according to claim 7, culturing the host cell under conditions for expression of the optical probe, and isolating the optical probe or fusion polypeptide.

9. Use of the optical probe according to any one of claims 2-4, the nucleic acid sequence according to claim 5, the nucleic acid construct according to claim 6, or the host cell according to claim 7 in detecting arginine in a sample, screening compounds, or localizing arginine inside/outside cells,
preferably,
the detection of arginine in a sample comprises the steps of: contacting the optical probe or the host cell with the sample, detecting the optical change of the optically active polypeptide, and quantifying arginine in the sample based on the optical change of the optically active polypeptide,
the screening of compounds comprises the steps of: contacting the optical probe or the host cell with a candidate compound in a system containing arginine, detecting the optical change of the optically active polypeptide, and screening the compound based on the optical change of the optically active polypeptide,
the localization of arginine inside/outside cells comprises the steps of: contacting a system containing arginine with the optical probe or the host cell, and detecting the optical change of the optically active polypeptide.

10. A detection kit, comprising:
(1) the optical probe according to any one of claims 2-4,
(2) the nucleic acid sequence according to claim 5,
(3) the nucleic acid construct according to claim 6, or
(4) the cell according to claim 7, and
other reagents required for detecting arginine using the optical probe,
preferably, the detection kit further comprises one or more reagents selected from: buffers, culture media, arginine standards.
